(19) Europäisches Patentamt
European Patent Office
Office européen des brevets

(11) Publication number: **0 097 056 A2**

(12) # EUROPEAN PATENT APPLICATION

(21) Application number: 83303436.6

(22) Date of filing: 14.06.83

(51) Int. Cl.³: C 07 C 103/737, C 07 C 121/43, C 07 D 295/18, C 07 D 307/52, A 01 N 37/30, A 01 N 37/34, A 01 N 43/08, A 01 N 43/84

(30) Priority: 14.06.82 JP 100773/82
18.03.83 JP 44460/83

(43) Date of publication of application: 28.12.83 Bulletin 83/52

(84) Designated Contracting States: BE CH DE FR GB IT LI LU NL SE

(71) Applicant: NIPPON KAYAKU KABUSHIKI KAISHA, 2-1, Marunouchi-1-chome Chiyoda-ku, Tokyo 100 (JP)

(72) Inventor: Yanagi, Mikio, 178-28, Ouaza-Idoki, Ageo-shi Saitama-ken (JP)
Inventor: Yamada, Osamu, 473-5, Mukouyama, Ageo-shi Saitama-ken (JP)
Inventor: Kobayashi, Takanori, 422, Ouaza-Obashira, Chichibu-shi Saitama-ken (JP)
Inventor: Futatsuya, Fumio, 9-306, 710-50, Ouaza-Higashiarai, Omiya-shi Saitama-ken (JP)

(74) Representative: Woods, Geoffrey Corlett et al, J.A. KEMP & CO. 14 South Square Gray's Inn, London WC1R 5EU (GB)

(54) **Herbicidal N-substituted-3,4,5,6-tetrahydrophthalamic acid derivatives.**

(57) N-substituted-3,4,5,6-tetrahydrophthalamic acid derivatives of the formula:

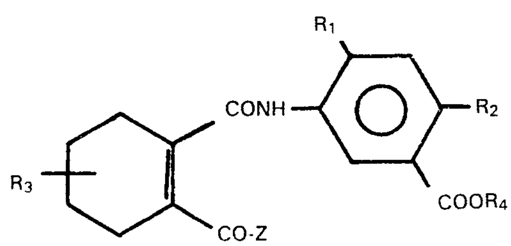

(1)

wherein $R_1$ is hydrogen or halogen; $R_2$ is halogen; $R_3$ is hydrogen or $C_1$-$C_4$ alkyl; $R_4$ is hydrogen or $C_1$-$C_8$-alkyl which may be substituted by halogen or $C_1$-$C_4$ alkoxy; and Z is hydroxy, $C_1$-$C_8$-primary or secondary alkylamino, $C_2$-$C_5$-alkenyl amino, $C_3$-$C_7$-cycloalkylamino, furfurylamino, benzylamino, morpholino which may be substituted by methyl, or $C_2$-$C_4$-alkylamino which may be substituted by phenyl, $C_1$ or $C_2$-alkylamino, $C_1$-$C_4$-alkoxy, $C_1$-$C_4$-alkylthio or cyano; and alkali metal salts thereof: are useful as herbicides.

EP 0 097 056 A2

0097056

# DESCRIPTION

## TITLE: HERBICIDAL N-SUBSTITUTED-3,4,5,6-TETRAHYDRO-PHTHALAMIC ACID DERIVATIVES

The present invention relates to an N-substituted-3,4,5,6-tetrahydrophthalamic acid derivative of the formula:

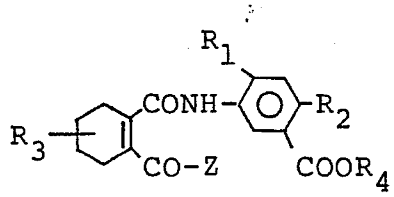

(1)

wherein $R_1$ is hydrogen or halogen, $R_2$ is halogen, $R_3$ is hydrogen or lower alkyl, $R_4$ is hydrogen, $C_1 \sim C_8$-alkyl which may have halogen or lower alkoxy and Z is hydroxy; $C_1 \sim C_8$-primary or secondary alkylamino: $C_2 \sim C_5$-alkenyl amino; $C_3 \sim C_7$-alicyclic alkylamino; $C_2 \sim C_4$-alkylamino which may have phenyl, furfuryl, $C_1 \sim C_2$-alkylamino, $C_1 \sim C_4$-alkoxy, $C_1 \sim C_4$-alkylthio or cyano; morpholino; morpholino which may have methyl; or alkali metal salt of hydroxy and a herbicidal composition containing said derivative as effective component, and processes for the production thereof, a herbicidal composition

containing said compound as effective component and further to a method of killing weeds using said compound.

It is known that some N-phenyl-3,4,5,6-tetrahydrophthalamic acids have herbicidal activity. (Japanese Patent Kokai No. 44425/1973, Japanese Patent Kokai No. 96722/1973 and Japanese Patent Kokai No. 431/1974).

The present inventors found that the compound of the formula (1) shows remarkably strong herbicidal activity as compared with known compounds of the above Japanese Patent Kokais. Furthermore, the present inventors found that the compound of the formula (1) has very low phytotoxicity against crops and, therefore, they become practical herbicide.

The compound of the formula (1) exhibits excellent herbicidal effect in a paddy field at a low dosage not only against annual weeds such as barnyard grasses and broadleaf weeds, but also against perennial weeds such as mizugayatsuri, bulrush, water chestnut, needle spikerush and arrowhead. The compound of the formula (1) also shows a good herbicidal effect by both pre- and post-emergence treatments in an up-land, especially against broadleaf weeds as those of amaranth, goosefoot and buckweat families at a low dosage.

On the other hand, the compound of the formula (1) is hardly phytotoxic to crops such as rice, wheat, oat, corn, soybean, cotton and sunflower.

The compound of the formula (1) of the present invention can be prepared by the processes described in a), b) and c) below.

a) The compound of the formula (1) wherein Z is hydroxy [hereinafter called "the compounds of the formula (4)"] can be obtained as follows:
3,4,5,6-tetrahydrophthalic anhydride of the formula (2) is reacted with m-aminobenzoic acid derivatives of the formula (3) in a proper solvent at a temperature of from room temperature to 120°C, preferably from room temperature to 80°C for a period of from 30 min. to several hrs. to produce the compound of the formula (4).

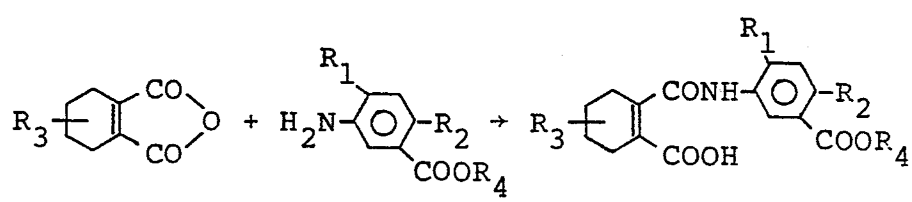

(2) (3) (4)

wherein $R_1$, $R_2$, $R_3$ and $R_4$ are defined as above.

b) The compound of formula (1) wherein Z is alkali metal salt of hydroxy is obtained by reacting the compound of the formula (4) with alkali metal hydroxide such

as sodium hydroxide and potassium hydroxide by an ordinary method.

c) The compound of the formula (1) wherein Z is hydroxy; $C_1$~$C_8$-primary or secondary alkylamino; $C_2$~$C_5$-alkenylamino; $C_3$~$C_7$-alicyclic alkylamino; $C_2$~$C_4$-alkylamino which may have phenyl, furfuryl, $C_1$~$C_2$-alkylamino $C_1$~$C_4$-alkoxy, $C_1$~$C_4$-alkylthio or cyano; morpholino; morpholino which may have methyl [hereinafter called "the compound of the formula (8)"] is obtained by heating the compound of the formula (4) in the presence of a proper dehydrating agent in a proper solvent at a temperature from room temperature to 100°C.

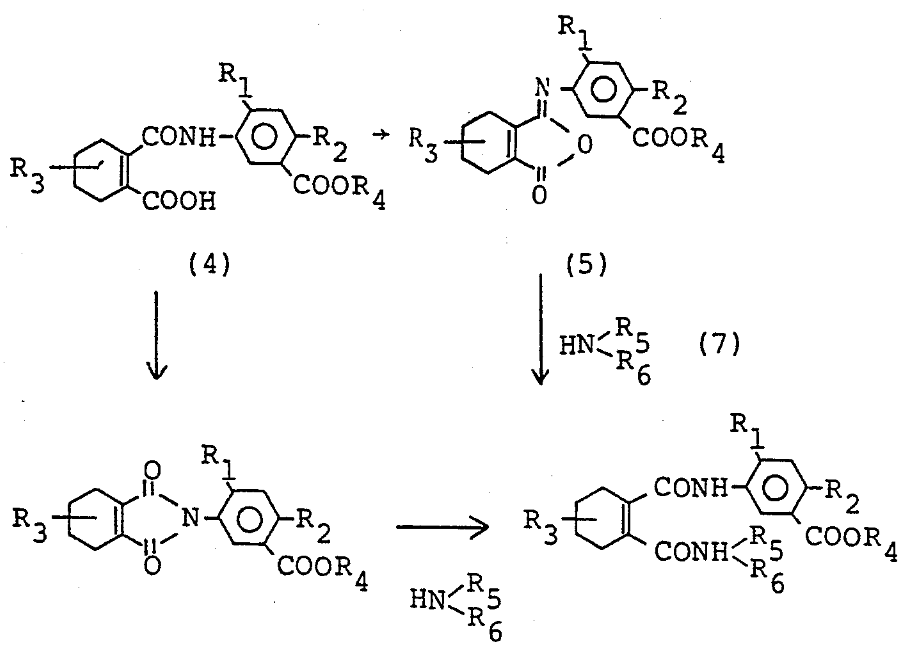

(4) (5) (7)

(6) (7) (8)

wherein $R_1$, $R_2$, $R_3$, $R_4$ are defined as above, $R_5$ and $R_6$ are defined after.

The compound of the formula (6) is obtained by heating the compound of the formula (4) in a proper solvent, if necessary in the presence of an acid catalyst at a temperature of from 50°C to 150°C.

The compound of the formula (8) is obtained by reacting the compound of the formula (5) or (6) with the compound of the formula (7) wherein $R_5$ is hydrogen or lower alkyl, $R_6$ is hydrogen; $C_1 \sim C_8$-alkyl; $C_2 \sim C_5$-alkenyl, $C_3 \sim C_7$ alicyclic alkyl; $C_2 \sim C_4$-alkyl which may have phenyl, furfuryl, $C_1 \sim C_2$-alkylamino, $C_1 \sim C_4$-alkoxy $C_1 \sim C_4$-alkylthio or cyano; or $R_5$ may together with $R_6$ make morpholino which may have methyl, preferably at a temperature of from room temperature to 120°C.

As examples of the dehydrating agents, there can be mentioned carbodiimide derivatives such as dicyclohexylcarbodiimide, diethylcarboxydiimide, mixture of a base and an acid halogenating agent or acylating agent.

As examples of the bases, there can be mentioned an aliphatic, aromatic or heterocyclic tertiary amines such as triethyl amine, dimethyl aniline or pyridine, and carbonates or hydrogen carbonates of alkali metal such as sodium carbonate, potassium carbonate and sodium hydrogen carbonates.

As examples of the acid halogenating agent there can be mentioned thionyl chloride or phosphorus oxychloride.

As examples of the acylating agent there can be mentioned an acid anhydride of an organic carboxylic acid or chlorocarbonic acid ester such as acetic acid anhydride or methyl chlorocarbonate.

As examples of the proper solvents, there can be mentioned lower aliphatic acids (acetic acid, propionic acid), aromatic compounds (toluene, xylene, chlorobenzene), halogenated hydrocarbons (chloroform, carbon tetrachloride, perchloroethylene), alcohols (methanol, ethanol), ethers (diethylether, dioxane tetrahydrofurane), ketones (acetone, methylethylketone) and water.

As examples of the acid catalysts, there can be mentioned p-toluenesulfonic acid, sulfuric acid and hydrogen chloride.

As examples of the halogen in $R_1$, $R_2$ and $R_4$ there can be mentioned chloro, bromo or fluoro.

As examples of the lower alkyls in $R_3$, there can be mentioned methyl, ethyl, propyl or butyl.

As examples of $C_1 \sim C_8$ - alkyls in $R_4$, there can be mentioned methyl, ethyl, n-propyl, i-propyl, n-butyl, sec-butyl, tert-butyl, n-pentyl, neopentyl, n-hexyl, i-hexyl, n-heptyl, n-octyl or 2-ethylhexyl.

As examples of $C_1 \sim C_8$-alkyls which are substituted by halogen, there can be mentioned chloromethyl, chloroethyl or 1-fluoromethyl-2-fluoropropyl.

As examples of $C_1 \sim C_8$-alkyls which may have lower alkoxy, there can be mentioned methoxyethyl, ethoxyethyl or methoxybutyl.

As the examples of $C_1 \sim C_8$-primary or secondary alkylamino there can be mentioned methylamino, ethylamino, n-propylamino, i-propylamino, n-butylamino, i-butylamino, sec-butylamino, tert-butylamino, i-amylamino, n-hexylamino, n-heptylamino, n-octylamino, dimethylamino, diethylamino, dipropylamino, isobutylamino, methylethylamino, ethylbutylamino or propylbutylamino.

As examples of $C_2 \sim C_5$-alkenylamino, there can be mentioned allylamino or allylmethylamino.

As examples of $C_3 \sim C_7$-alicyclic alkylamino, there can be mentioned cyclohexylamino.

As examples of $C_2 \sim C_4$-alkylamino which may have phenyl there can be mentioned 1,1-dimethyl-1-phenylamino or benzylamino. As examples of $C_2 \sim C_4$ alkylamino which has furfuryl, there can be mentioned $-NHCH_2-$ 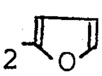 .

As example of $C_2 \sim C_4$-alkylamino which has $C_1 \sim C_2$-alkylamino, there can be mentioned $-NH(CH_2)_3N<^{CH_3}_{CH_3}$ .

As examples of $C_2$∿$C_4$-alkylaminos which have $C_1$∿$C_4$-alkoxy, there can be mentioned 2-ethoxyethylamino, 3-methoxypropylamino, 4-methoxybutylamino, 5-methoxybutylamino, 2-ethoxyethylamino, 3-ethoxypropylamino, 2-propoxyethylamino, 3-propoxypropylamino, 2-butoxyethylamino or 1-ethyl-2-methoxyethylamino.

As examples of $C_2$∿$C_4$-alkylaminos which have $C_1$∿$C_4$-alkylthio, there can be mentioned $-NHCH_2CH_2CH_2SCH_3$ or $-NHCH_2CH_2CH_2SC_2H_5$.

As example of $C_2$∿$C_4$-alkylamino which have cyano, there can be mentioned cyanoethyl.

As examples of the alkali metal salts of hydroxy, there can be mentioned -ONa and -OK.

As preferable compounds in the present invention, there can be mentioned those of the formula (1) wherein $R_1$ is chloro or fluoro, $R_2$ is chloro or bromo, $R_3$ is hydrogen or methyl, $R_4$ is $C_2$∿$C_3$-alkyl, Z is hydroxy, $C_1$∿$C_3$-alkylamino or $C_1$∿$C_3$-alkoxy-$C_2$∿$C_3$-alkylamino.

As more preferable compounds in the present invention, there can be mentioned those of the formula (1) wherein $R_1$ is fluoro, $R_2$ is chloro or bromo, $R_3$ is hydrogen, $R_4$ is i-propyl, Z is $C_1$∿$C_3$ alkylamino or $C_1$∿$C_3$-alkoxy-$C_2$∿$C_3$ alkylamino. Examples of such compounds include those which are given in the table below

as compounds Nos. 43, 45, 57, 62∿65, 73, 74, 76∿81, 83, 84, 88, 89, 91∿94, 96, 97, 111.

The present invention will be illustrated in the following examples.

Synthesis Example 1: N-(4-chloro-3-isopropoxycarbonylphenyl)-3,4,5,6-tetrahydrophthalamic acid (No. 13).

24.5 g (0.16 mol) of 3,4,5,6-tetrahydrophthalic anhydride were dissolved in 200 ml of ethyl ether.

Then 32.8 g (0.15 mol) of 4-chloro-3-isopropoxycarbonylaniline were added slowly to the solution under stirring and the reaction was effected at 20 to 28°C for 2 hrs.

The resulting crystals were filtered and washed with ether and acetone and 50 g of white crystals were obtained.

M.p. 133 to 134°C

| | | | |
|---|---|---|---|
| Elementary analysis: $C_{18}H_{20}Cl\ N\ O_5$ | | | |
| Calculated | : C:59.10 | H:5.51 | N:3.83 |
| Found | : C:59.15 | H:5.48 | N:3.86 |

Synthesis Example 2: N-(4-bromo-2-fluoro-5-isopropoxycarbonylphenyl)-3,4,5,6-tetrahydrophthalamic acid (No. 61)

80 ml of ether were added to 15.2 g (0.1 mol) of 3,4,5,6-tetrahydrophthalic anhydride and 27.6 g

(0.1 mol) of 4-bromo-2-fluoro-5-isopropoxycarboxyaniline were added to the mixture slowly at room temperature.

After the reaction was effected for 2.5 hrs at 30 to 40°C, 50 ml of an aqueous solution containning 11 g (0.1 mol) of sodium carbonate was added to the reaction mixture at 15 to 20°C and the reaction mixture was stirred for 2 hrs.

The reaction mixture was subjected to extraction using toluene, and the toluene layer was washed with an aqueous solution of sodium carbonate. The water layers were collected and acidified by hydrochloric acid aqueous solution to produce 35 g of pale yellow crystals.

M.p. 94 to 96°C

Elementary analysis: $C_{18}H_{19}BrFNO_5$

| | | | |
|---|---|---|---|
| Calculated | : C:50.48 | H:4.47 | N:3.27 |
| Found | : C:50.55 | H:4.49 | N:3.30 |

Synthesis Example 3: N-ethyl-N'-(4-chloro-3-isopropoxycarbonylphenyl)-3,4,5,6-tetrahydrophthalamide (No. 14)

27.22 g of N-(4-chloro-3-isopropoxycarbonylphenyl)-3,4,5,6-tetrahydrophthalamic acid (No. 13) which were obtained according to Synthesis Example 1 were suspended in 200 ml of benzene and 18.42 g (0.089 mol) of

dicyclohexylcarbodiimide were added to the suspension, and the whole was stirred at room temperature for 2 hrs. The residue which did not dissolve in benzene were removed by filtration, the filtrate was concentrated under reduced pressure to produce an oil. A small amount of n-hexane were added to the oil and the whole was allowed to stand to precipitate the crystals. 21.2 g of N-(4-chloro-3-isopropoxycarbonylphenyl)-3,4,5,6-tetrahydroisophthalimide were obtained after recrystallization of the precipitated crystals.
M.p. 100 to 102°C.

To 3 g (0.009 mol) of thus obtained compound were added 20 ml of acetone, 1 g (0.015 mol) of 70% ethylamine aqueous solution was added dropwise thereto and the reaction was effected at room temperature for 2 hrs. to produce crystals. The crystals were filtered and washed with n-hexane to obtain 2.8 g of intended white crystals. M.p. 161 to 163°C.

Elementary analysis: $C_{20}H_{25}ClN_2O_4$

| | |
|---|---|
| Calculated | : C:61.14 H:6.41 N:7.13 |
| Found | : C:61.22 H:6.43 N:7.18 |

Synthesis Example 4: N-methyl-N'-(4-bromo-2-fluoro-5-isopropoxycarbonylphenyl)-3,4,5,6-tetrahydrophthalamide (No. 62)

30 g (0.07 mol) of N-(4-bromo-2-fluoro-5-isopropoxy-carboxyphenyl)-3,4,5,6-tetrahydrophthalamic acid (No. 64) which had been obtained by Synthesis Example 2 were suspended in 120 ml of benzene, and 15.9 g of dicyclohexylcarboxydiimide were added to the suspension and the suspension was stirred for 2 hrs. at room temperature.

Then the residue which was not dissolved in benzene was removed by filtration and the filtrate was concentrated under a reduced pressure to obtain 30 g of crystaline N-(4-bromo-2-fluoro-5-isopropoxycarbonylphenyl)-3,4,5,6-tetrahydrophthalimide.

The pale yellow crystals which were obtained by recrystallization had m.p. of 81 to 82°C.

To 1.6 g (0.004 mol) of thus obtained compound, added 30 ml of ether and 0.3 g (0.004 mol) of methylamine using 40% of an aqueous methylamine solution was added dropwise to the mixture at room temperature under stirring for 30 minutes.

Then the precipitated crystals were obtained by filtration and washed with ether and 13.7 g of the intended compound. M.p. 122.5 to 123.5°C.

Elementary analysis: $C_{19}H_{22}BrFN_2O_4$

| | | | |
|---|---|---|---|
| Calculated | : C:51.71 | H:5.02 | N:6.35 |
| Found | : C:51.80 | H:5.08 | N:6.40 |

Synthesis Example 5: N-methyl-N'-(4-chloro-2-fluoro-5-isopropoxycarbonylphenyl)-3,4,5,6-tetrahydrophthalamide (No. 57)

4 g of N-(4-chloro-2-fluoro-5-isopropoxycarbonylpheny 3-4,5,6-tetrahydroisophthalimide were dissolved in 70 ml of tetrachloromethane and to the mixture, 0.93 g of 40% of an aqueous methylamine solution was added dropwise. After the stirring for 30 minutes, the crystals were precipitated and the crystals were filtered and washed with n-hexane to produce 3.8 g of the intended compound (white crystals). M.p.: 147 to 149°C.

Elementary analysis: $C_{19}H_{22}ClFN_2O_4$

| | | | |
|---|---|---|---|
| Calculated | : C:57.50 | H:5.59 | N:7.06 |
| Found | : C:57.72 | H:5.61 | N:7.10 |

Synthesis Example 7: N-methyl-N'-(4-chloro-2-fluoro-5-isopropoxycarbonylphenyl)-3,4,5,6-tetrahydrophthalimide (No. 57)

To 33.5 g of 3,4,5,6-tetrahydrophthalic anhydride, 200 ml of toluene, 51 g of 5-amino-2-chloro-4-fluorobenzoic acid isopropylester and 1.8 g of p-toluenesulfonic acid were added. The mixture was refluxed to effect dehydration reaction.

After the reaction, ethylacetate was added to the reaction mixture. Then the reaction mixture was washed with water and concentrated to precipitate the

crystals. The crystals were subjected to recrystallization to obtain 73.5 g of white crystaline N-(4-chloro-2-fluoro -5-isopropoxycarbonylphenyl)-3,4,5,6-tetrahydrophthalimide (m.p. 74-5°C).

4 g of thus obtained compound were dissolved in 30 ml of benzene, and then 0.97 g of 40% of an aqueous methylamine solution was added dropwise to the mixture and the mixture was stirred for 30 minutes. The precipitated crystals were filtered and washed with benzene to produce 3.8 g of white crystals having m.p. of 147 to 149°C. The data concerning IR and NMR of the compound were identical with the data of the compound obtained by Synthesis Example 5.

Examples of those compounds obtained by the above mentioned method are given in Table 1 below.

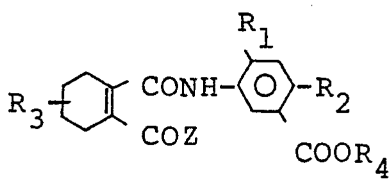

Table 1

| No. | $R_1$ | $R_2$ | $R_3$ | $R_4$ | Z | M.P. (°C) or Refractive index | Appearance |
|---|---|---|---|---|---|---|---|
| 1 | H | F | H | H | OH | 226-230 | White crystal |
| 2 | H | F | H | $C_2H_5$ | OH | 115-116 | White crystal |
| 3 | H | Cl | H | $CH_3$ | OH | 123-125 | White crystal |
| 4 | H | Cl | H | $C_2H_5$ | $NHCH_3$ | 103-106 | White crystal |
| 5 | H | Cl | H | $C_2H_5$ | $NHC_2H_5$ | 130-133 | White crystal |
| 6 | H | Cl | H | $C_2H_5$ | $NHC_3H_7$(i) | 114-115 | White crystal |
| 7 | H | Cl | H | $C_2H_5$ | $NHC_3H_7$(i) | 167-170 | White crystal |
| 8 | H | Cl | H | $C_2H_5$ | $NHCH_2CH=CH_2$ | 120-123 | White crystal |
| 9 | H | Cl | H | $C_2H_5$ | $NHC_4H_9$(n) | 112-114 | White crystal |
| 10 | H | Cl | H | $C_2H_5$ | $NHC_4H_9$(i) | 114-117 | White crystal |
| 11 | H | Cl | H | $C_2H_5$ | $NHC_4H_9$(s) | 171-174 | White crystal |
| 12 | H | Cl | H | $C_2H_5$ | NH- 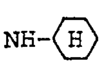 | 164-167 | White crystal |
| 13 | H | Cl | H | $C_3H_7$(i) | OH | 133-134 | White crystal |
| 14 | H | Cl | H | $C_3H_7$(i) | $NHC_2H_5$ | 161-163 | White crystal |

| No. | $R_1$ | $R_2$ | $R_3$ | $R_4$ | Z | M.P. (°C) or Refractive index | Appearance |
|---|---|---|---|---|---|---|---|
| 15 | H | Cl | H | $C_3H_7$(i) | $NHC_3H_7$(n) | 126-129 | White crystal |
| 16 | H | Cl | H | $C_3H_7$(i) | $NHC_3H_7$(i) | 161-163 | White crystal |
| 17 | H | Cl | H | $C_3H_7$(i) | $NHC_4H_9$(t) | 205-208 | White crystal |
| 18 | H | Cl | H | $C_3H_7$(i) | $NHC_8H_{17}$(n) | 115-117 | White crystal |
| 19 | H | Cl | H | $C_3H_7$(i) | 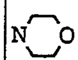 | 145-146 | White crystal |
| 20 | H | Cl | H | $C_3H_7$(i) | 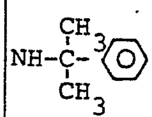 | 225-228 | White crystal |
| 21 | H | Cl | H | $C_4H_9$(s) | $NHC_2H_5$ | 136-139 | White crystal |
| 22 | H | Cl | H | $C_4H_9$(s) | $NHC_3H_7$(i) | 148-151 | White crystal |
| 23 | H | Cl | H | $C_4H_9$(s) | $NHC_3H_7$(n) | 143-145 | White crystal |
| 24 | H | Cl | H | $C_4H_9$(s) | $NHC_6H_{13}$(n) | 103-106 | White crystal |
| 25 | H | Cl | H | $C_2H_4OCH_3$ | OH | 97-98 | White crystal |
| 26 | H | Cl | H | $C_2H_4OCH_3$ | $NHCH_3$ | 114-117 | White crystal |
| 27 | H | Cl | H | $C_2H_4OCH_3$ | $NHC_2H_5$ | 140-143 | White crystal |
| 28 | H | Cl | H | $C_2H_4OCH_3$ | $NHC_3H_7$(n) | 128-132 | White crystal |
| 29 | H | Cl | H | $CH-CH_2F$<br>$CH_2F$ | $NHCH_3$ | 128-131 | White crystal |
| 30 | H | Cl | H | $CH_2CH(CH_2)_3CH_3$<br>$C_2H_5$ | $NHC_3H_7$(n) | $n_D^{25}$ 1.5365 | Yellow oil |
| 31 | H | Cl | 4- or 5-$CH_3$ mix. | $C_3H_7$(i) | OH | 123-124 | White crystal |

| No. | $R_1$ | $R_2$ | $R_3$ | $R_4$ | Z | M.P. (°C) or Refractive index | Appearance |
|---|---|---|---|---|---|---|---|
| 32 | H | Cl | 4- or 5-$CH_3$ mix. | $C_3H_7$(i) | $NHC_2H_5$ | 130-132 | White crystal |
| 33 | H | Cl | 3- or 6-$CH_3$ mix. | $C_3H_7$(i) | $NHC_2H_5$ | 165-166.5 | White crystal |
| 34 | Cl | Cl | H | $CH_3$ | OH | 124-126 | White crystal |
| 35 | Cl | Cl | H | $CH_3$ | $NHCH_3$ | 170-174 dec | White crystal |
| 36 | Cl | Cl | H | $CH_3$ | $NHC_2H_5$ | 171-172 dec | White crystal |
| 37 | Cl | Cl | H | $CH_3$ | $NHCH_2CH=CH_2$ | 169 dec | White crystal |
| 38 | Cl | Cl | H | $CH_3$ | $NHC_4H_9$(s) | 182-183 | White crystal |
| 39 | Cl | Cl | H | $CH_3$ | $NH-(CH_2)_3N\langle^{CH_3}_{CH_3}$ | 99-102 | White crystal |
| 40 | Cl | Cl | H | $CH_3$ | $NHC_3H_7$(i) | 181-182 | White crystal |
| 41 | Cl | Cl | H | $CH_3$ | $NHC_4H_9$(i) | 168.5-170 | White crystal |
| 42 | Cl | Cl | H | $C_3H_7$(i) | OH | 109-110 | White crystal |
| 43 | Cl | Cl | H | $C_3H_7$(i) | $NHC_2H_5$ | 139-141 | White crystal |
| 44 | Cl | Cl | H | $C_3H_7$(i) | $NHC_3H_7$(n) | 123-128 | White crystal |
| 45 | Cl | Cl | H | $C_3H_7$(i) | $NHC_3H_7$(i) | 142-145 | White crystal |
| 46 | Cl | Cl | H | $C_3H_7$(i) | $NHCH_2CH=CH_2$ | 134.5-135 | White crystal |
| 47 | Cl | Cl | H | $C_3H_7$(i) | $NHC_4H_9$(s) | 157-160 | White crystal |
| 48 | Cl | Cl | H | $C_3H_7$(i) | NH-⟨H⟩ | 183-185 | White crystal |
| 49 | H | Br | H | $C_2H_5$ | OH | 64-66 | Pale brown crystal |

| No. | $R_1$ | $R_2$ | $R_3$ | $R_4$ | Z | M.P. (°C) or Refractive index | Appearance |
|---|---|---|---|---|---|---|---|
| 50 | H | Br | H | $C_3H_7$(i) | $N\langle^{CH_3}_{CH_3}$ | 120-122 | White crystal |
| 51 | H | Br | 4- or 5-$CH_3$ | $C_3H_7$(i) | OH | 123-125 | Pale yellow crystal |
| 52 | H | Br | 4- or 5-$CH_3$ | $C_3H_7$(i) | $NHC_2H_5$ | 121-123 | White crystal |
| 53 | Cl | Cl | 4- or 5-$CH_3$ | $C_3H_7$(i) | OH | 114-115 | White crystal |
| 54 | Cl | Cl | 4- or 5-$CH_3$ | $C_3H_7$(i) | $NHC_2H_5$ | 164-167 | White crystal |
| 55 | Cl | Cl | 4- or 5-$CH_3$ | $C_3H_7$(i) | $NHC_3H_7$(i) | 158-162 | White crystal |
| 56 | F | Cl | H | $C_3H_7$(i) | OH | 91-93 | White crystal |
| 57 | F | Cl | H | $C_3H_7$(i) | $NHCH_3$ | 147-149 | White crystal |
| 58 | F | Cl | H | $C_3H_7$(i) | $NHC_3H_7$(n) | 136-137 | White crystal |
| 59 | F | Cl | H | $C_3H_7$(i) | 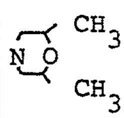 | 110-113 | White crystal |
| 60 | F | Br | H | H | OH | 239 dec | White crystal |
| 61 | F | Br | H | $C_3H_7$(i) | OH | 94-96 | Pale yellow crystal |
| 62 | F | Br | H | $C_3H_7$(i) | $NHCH_3$ | 122.5-123.5 | White crystal |
| 63 | F | Br | H | $C_3H_7$(i) | $NHC_2H_5$ | 120-121 | White crystal |
| 64 | F | Br | H | $C_3H_7$(i) | $NHC_3H_7$(n) | 122-123 | White crystal |
| 65 | F | Br | H | $C_3H_7$(i) | $NHC_3H_7$(i) | 128-129 | White crystal |
| 66 | F | Br | H | $C_3H_7$(i) | $NHC_4H_9$(n) | 115-115.5 | White crystal |
| 67 | F | Br | H | $C_3H_7$(i) | $NHC_4H_9$(s) | 147.5-148 | White crystal |

| No. | $R_1$ | $R_2$ | $R_3$ | $R_4$ | Z | M.P. (°C) or Refractive index | Appearance |
|---|---|---|---|---|---|---|---|
| 68 | F | Br | H | $C_3H_7$(i) | $NHC_4H_9$(t) | 69.5-70.5 | White crystal |
| 69 | F | Br | H | $C_3H_7$(i) | $NHCH_2$- 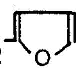 | 106-108 | White crystal |
| 70 | F | Br | H | $C_3H_7$(i) | $NHCH_2CH=CH_2$ | 113.5-115 | White crystal |
| 71 | F | Cl | 4- or 5-$CH_3$ | $C_3H_7$(i) | OH | 110-113 | White crystal |
| 72 | F | Cl | 4- or 5-$CH_3$ | $C_3H_7$(i) | $NHCH_3$ | 112-115 | White crystal |
| 73 | F | Cl | 4- or 5-$CH_3$ | $C_3H_7$(i) | $NHC_2H_5$ | 170-172 | White crystal |
| 74 | F | Cl | 4- or 5-$CH_3$ | $C_3H_7$(i) | $NHC_3H_7$(n) | 146-149 | White crystal |
| 75 | F | Br | H | $C_3H_7$(i) | ONa | > 260 | Pale yellow crystal |
| 76 | F | Cl | H | $C_3H_7$(i) | $NH-C_2H_4OCH_3$ | 87-92 | White crystal |
| 77 | F | Cl | H | $C_3H_7$(i) | $NHC_2H_4OC_2H_5$ | 127-129 | White crystal |
| 78 | F | Br | H | $C_3H_7$(i) | $NHC_2H_4OCH_3$ | 114-115.5 | White crystal |
| 79 | F | Br | H | $C_3H_7$(i) | $NH-C_2H_4OC_2H_5$ | 104-107 | White crystal |
| 80 | F | Cl | H | $C_3H_7$(i) | $NH-(CH_2)_3OC_2H_5$ | 75-80 | White crystal |
| 81 | F | Cl | H | $C_3H_7$(i) | $NH(CH_2)_3OC_3H_7$(i) | 78-83 | White crystal |
| 82 | F | Cl | H | $C_3H_7$(i) | $NH-CHCH_2OCH_3$<br>$C_2H_5$ | 157-158 | White crystal |
| 83 | F | Br | H | $C_2H_5$ | $NH(CH_2)_2OCH_3$ | 127~128 | White crystal |
| 84 | F | Br | H | $C_2H_5$ | $NH(CH_2)_2OC_2H_5$ | 120~121 | White crystal |
| 85 | H | Cl | H | $C_3H_7$(i) | $NH(CH_2)_2OCH_3$ | 137~138 | White crystal |

| No. | $R_1$ | $R_2$ | $R_3$ | $R_4$ | Z | M.P. (°C) or Refractive index | Appearance |
|---|---|---|---|---|---|---|---|
| 86 | H | Cl | H | $C_3H_7$(i) | $NH(CH_2)_2OC_2H_5$ | 117~119 | White crystal |
| 87 | H | Cl | H | $C_3H_7$(i) | $NHCHCH_2OCH_3$ with $C_2H_5$ on CH | 121~125 | White crystal |
| 88 | F | Cl | 4-$CH_3$ or 5-$CH_3$ mix. | $C_3H_7$(i) | $NH(CH_2)_2OCH_3$ | 102~105 | White crystal |
| 89 | F | Cl | 4-$CH_3$ or 5-$CH_3$ mix. | $C_3H_7$(i) | $NH(CH_2)_2OC_2H_5$ | 106~110 | White crystal |
| 90 | F | Br | 4-$CH_3$ or 5-$CH_3$ mix. | $C_3H_7$(i) | $NHCHCH_2OCH_3$ with $C_2H_5$ on CH | 141~145 | White crystal |
| 91 | F | Br | 4-$CH_3$ or 5-$CH_3$ mix. | $C_3H_7$(i) | $NH(CH_2)_2OCH_3$ | 103~108 | White crystal |
| 92 | F | Br | 4-$CH_3$ or 5-$CH_3$ mix. | $C_3H_7$(i) | $NH(CH_2)_2OC_2H_5$ | 99~103 | White crystal |
| 93 | Cl | Cl | H | $C_3H_7$(i) | $NH(CH_2)_2OCH_3$ | 114~118 | White crystal |
| 94 | F | Cl | H | $CHCH_2-CHCH_3$ with $CH_3$, $CH_3$ | $NH(CH_2)_2OCH_3$ | 102~106 | White crystal |
| 95 | F | Cl | H | $C_3H_7$(i) | $NH(CH_2)_2OC_3H_7$(i) | 142-143 | White crystal |
| 96 | F | Cl | H | $C_3H_7$(i) | $NH(CH_2)_2OC_3H_7$(n) | 129-131 | White crystal |
| 97 | F | Cl | 4-$CH_3$ or 5-$CH_3$ mix. | $C_3H_7$(i) | $NH(CH_2)_2OC_3H_7$(n) | 145-146 | White crystal |
| 98 | F | Cl | 4-$CH_3$ or 5-$CH_3$ mix. | $C_3H_7$(i) | $NH(CH_2)_2OC_3H_7$(i) | 130-132 | White crystal |
| 99 | F | Cl | H | $C_3H_7$(i) | $NHC_4H_9$(sec) | 163-164 | White crystal |

| No. | $R_1$ | $R_2$ | $R_3$ | $R_4$ | Z | M.P. (°C) or Refractive index | Appearance |
|---|---|---|---|---|---|---|---|
| 100 | F | Cl | H | $C_3H_7$(i) | $NHCH_2CH_2CH_2OCH_3$ | 101-105 | White crystal |
| 101 | F | Cl | 4- or 5-$CH_3$ mix. | $C_3H_7$(i) | $NHCH_2CH_2CH_2OCH_3$ | 127-129 | White crystal |
| 102 | F | Cl | H | $C_3H_7$(i) | $NHCH_2\text{-}CH<^{OCH_3}_{OCH_3}$ | 113-114 | White crystal |
| 103 | F | Cl | 4- or 5-$CH_3$ mix. | $C_3H_7$(i) | $NHCH_2\text{-}CH<^{OCH_3}_{OCH_3}$ | 110-113 | White crystal |
| 104 | F | Cl | H | $C_3H_7$(i) | $NHCH_2CH<^{OC_2H_5}_{OC_2H_5}$ | 118-119 | White crystal |
| 105 | F | Cl | 4- or 5-$CH_3$ mix. | $C_3H_7$(i) | $NHCH_2CH<^{OC_2H_5}_{OC_2H_5}$ | 119-122 | white crystal |
| 106 | F | Cl | H | $C_3H_7$(i) | $NHCH_2CH_2CH_2SCH_3$ | 124-125 | White crystal |
| 107 | F | Cl | 4- or 5-$CH_3$ mix. | $C_3H_7$(i) | $NHCH_2CH_2CH_2SCH_3$ | 127-132 | White crystal |
| 108 | F | Cl | H | $C_3H_7$(i) | $NHCH_2CH_2CN$ | 154-156 | White crystal |
| 109 | Cl | Cl | H | $C_3H_7$(i) | $N<^{CH_3}_{CH_3}$ | 106-108 | White crystal |
| 110 | Cl | Cl | H | $C_3H_7$(i) | $N<^{C_2H_5}_{C_2H_5}$ | 96-97 | White crystal |
| 111 | F | Cl | H | $C_3H_7$(i) | $NHC_3H_7$(i) | 176-176.5 | White crystal |

The herbicidal composition of the present invention can be used either alone or in the form of a formulation according to the purpose of its use. To promote or secure the effect, it is mixed with adjuvants to make formulations such as dust, micro granule, granule, wettable powder, flowable suspension concentrates and emulsion by means of usual procedures. These formulations are used, at the time of practical application, in the form as they are or diluted with water to desired concentration.

Those adjuvants mentioned above include carriers (diluents), extending agents, emulsfiers, wetting agents, dispersing agents, fixing agents and disintegrators.

As liquid carriers there can be used water, aromatic hydrocarbons such as toluene and xylene, alcohols such as methanol, butanol and glycol, ketones such as acetone, amides such as dimethylformamide, sulfoxides such as dimethylsulfoxide, methylnaphthalene, cyclohexane, animal and vegetable oils, fatty acids and their esters, etc. As solid carriers are used clay, kaolin, talc, diatomaceous earth, silica, calcium carbonate, montmorillonite, bentonite, feldspar, quartz, alumina, sawdust, etc.

As emulsifiers or dispersing agents surfactants

are generally used. They include anionic, cationic, nonionic and amphoteric surfactants such as sodium salts of sulfated higher alcohol, steariltrimethylammonium chloride, polyoxyethylenealkylphenylether and lauryl betaine. Wetting agents include sodium alkylnaphthalene sulfonate and ammonium polyoxyethylenealkylphenylether sulfate, fixing agents include polyvinyl alcohol, polyvinyl acetate and CMC, and disintegrators include sodium lignin sulfonate and sodium salt of lauryl sulfate.

Any type of said formulations can not only used alone, but also may be mixed with fungicides, insecticides, plant growth regulators, acaricides, soil modifying agents or nematocides and further can be used in combination with fertilizers or other herbicides.

The content of a compound (acitive ingredient) of the present invention in the formulations varies with types of formulation, methods of application and other conditions, but generally it is 0.5 to 95 weight %, preferably 2 to 50 weight %, while the content of adjuvants is 5 to 99.5 weight %, preferably 50 to 98 weight %, though sometimes the compound can be used alone.

To be more precise, a preferable range of the content is shown as under.

| | Compound (weight %) | Adjuvant (weight %) |
|---|---|---|
| Dust | 0.5 - 10 | 90 - 99.5 |
| Emulsion | 20 - 80 | 20 - 80 |
| Wettable powder | 20 - 80 | 20 - 80 |
| Granule and micro granule | 0.5 - 20 | 80 - 99.5 |
| Flowable suspension concentrate | 20 - 80 | 20 - 80 |

A quantity to use of the formulations varies with kinds of the active ingredient and places of application, but generally it is within the range of 1 to 100 g, preferably 3 to 75 g, of the compound per are.

The active ingredient of the present invention can be used by applying directly to weeds or the locus of the weeds.

Detailed explanation will be made below on examples formulations of the present invention and there the word "part" means part by weight.

Formulation Example 1 : Emulsion

35 parts of a mixture (1 : 1) of xylene and methylnaphthalene are added to 50 parts of Compound No. 14 to dissolve and the solution is further mixed with

15 parts of a mixture (8 : 2) of polyoxyethylenealkylphenylether and calcium alkylbenzenesulfonate to obtain an emulsion. It is diluted with water to use in a concentration of 0.01 to 1%.

Formulation Example 2 : Dust

5 parts of Compound No. 57 are mixed with 95 parts of clay and pulverized to obtain a dust. It is directly used for dusting.

Formulation Example 3 : Wettable powder

50 parts of Compound No. 58 are mixed with 10 parts of diatomaceous earth and 32 parts of kaolin and further uniformly blended with 8 parts of a mixture of sodium laurylsulfate and sodium 2,2'-dinaphtylmethanesulfonate, and finely pulverized to obtain a wettable powder. It is used in the form of a suspension by diluting to a concentration of 0.06 to 1%.

Formulation Example 4: Granule

5 parts of a fine dust of Compound No. 62 are extended for coating on 94.5 parts of grains (16 to 32 mesh) of silica to obtain a granule, by using a methanol solution of 0.5 parts of polyvinyl polyacetate as a binding agent in a proper mixer. The granule is a scattered directly in up-land field and paddy field.

Formulation Example 5 : Flowable suspension concentrates

40 parts of a fine powder of Compound No.111, 10 parts of ethyleneglycolmonobutylether, 10 parts of a surfactant (mixture of trioxyalkylether, polyoxyethylene-nonylphenylether and sodium alkylarylsulfonate), 3 parts of colloidal aluminium silicate hydrate and 22 parts of water are uniformly mixed and further blended under stirring in a homomixer for 20 minutes to obtain a flowable suspension concentrate. It is diluted with water for use in a concentration of 0.02 to 1%.

The excellent herbicidal activity of a compound of the present invention will be illustrated in the following test examples.

Each test was carried out on 2-replication system and the test results are given in the average value.

Test Example 1 : Pre-emergence treatment in flooded condition

A fixed amount of paddy field soil was filled in each Wagner pot sized 1/5,000 are to provide a condition similar to a paddy field and there was sown a fixed amount of seeds of barnyard grass, monochoria, toothcup, false pimpernel, water wort and umbrella plant.

In addition tubers of arrowhead were buried 1

cm under the surface of soil at the rate of 3 pieces per pot and the pot was flooded with water 3 cm deep. Then the pot was applied with a diluted solution of the compound of the present invention at a rate of 6.25 to 50 g of the compound of the present invention per are.

After three days 3 pieces of rice seedlings (variety : Nihonbare) in 2.5-leaf stage were transplanted from a nursery to each pot. Thirty days after the treatment the herbicidal activity and the phytotoxicity against paddy rice were observed. The test results were classified on the following basis as shown in Table 3.

Herbicidal activity index:

| | |
|---|---|
| 5 | complete weeding |
| 4 | up to 80% weeding |
| 3 | up to 60% weeding |
| 2 | up to 40% weeding |
| 1 | up to 20% weeding |
| 0 | no effect |

Phytotoxicity index:

| | |
|---|---|
| - | no damage |
| + | slight damage |
| ++ | some damage |
| +++ | moderate damage |
| ++++ | heavy damage |
| x | complete death |

Table 2

Test Example 1: Pre-emergence treatment under flooded condition

| Compound No. | Dosage g/a | Herbicidal activity | | | | Phytotoxicity against paddy rice |
|---|---|---|---|---|---|---|
| | | Barnyard grass | Broad leaf (1) | Umbrella sedge (2) | Arrow head | |
| 2 | 25<br>12.5<br>6.25 | 4<br>3<br>2 | 5<br>5<br>5 | 5<br>5<br>5 | 5<br>2<br>2 | -<br>-<br>- |
| 4 | 25<br>12.5<br>6.25 | 5<br>5<br>4 | 5<br>5<br>5 | 5<br>5<br>5 | 3<br>3<br>3 | ±<br>-<br>- |
| 5 | 25<br>12.5<br>6.25 | 5<br>5<br>5 | 5<br>5<br>5 | 5<br>5<br>5 | 5<br>4.5<br>4 | +<br>±<br>- |
| 8 | 25<br>12.5<br>6.25 | 5<br>5<br>4 | 5<br>5<br>5 | 5<br>5<br>5 | 4<br>2<br>2 | ±<br>-<br>- |
| 9 | 25<br>12.5<br>6.25 | 5<br>5<br>5 | 5<br>5<br>5 | 5<br>5<br>5 | 5<br>5<br>5 | +<br>±<br>- |
| 10 | 25<br>12.5<br>6.25 | 5<br>5<br>5 | 5<br>5<br>5 | 5<br>5<br>5 | 5<br>4.5<br>2 | ++<br>±<br>± |
| 12 | 25<br>12.5<br>6.25 | 5<br>5<br>5 | 5<br>5<br>5 | 5<br>5<br>5 | 3<br>2<br>2 | +<br>±<br>- |
| 15 | 25<br>12.5<br>6.25 | 5<br>5<br>5 | 5<br>5<br>5 | 5<br>5<br>5 | 4<br>3<br>2 | ±<br>-<br>- |
| 23 | 25<br>12.5<br>6.25 | 5<br>5<br>5 | 5<br>5<br>5 | 5<br>5<br>5 | 4<br>3<br>2 | -<br>-<br>- |
| 24 | 25<br>12.5<br>6.25 | 5<br>5<br>4.5 | 5<br>5<br>5 | 5<br>5<br>5 | 5<br>5<br>5 | -<br>-<br>- |

| Compound No. | Dosage g/a | Herbicidal activity | | | | Phytotoxicity against paddy rice |
|---|---|---|---|---|---|---|
| | | Barnyard grass | Broad leaf (1) | Umbrella sedge (2) | Arrow head | |
| 27 | 25<br>12.5<br>6.25 | 5<br>2<br>2 | 5<br>5<br>5 | 5<br>5<br>5 | 5<br>5<br>5 | +<br>-<br>- |
| 29 | 25<br>12.5<br>6.25 | 5<br>4.8<br>4 | 5<br>5<br>5 | 5<br>5<br>5 | 5<br>2<br>1 | -<br>-<br>- |
| 31 | 25<br>12.5<br>6.25 | 5<br>5<br>4 | 5<br>5<br>5 | 5<br>5<br>5 | 5<br>5<br>3 | -<br>-<br>- |
| 35 | 25<br>12.5<br>6.25 | 5<br>5<br>4.5 | 5<br>5<br>5 | 5<br>5<br>5 | 5<br>4<br>3 | +<br>+<br>- |
| 37 | 25<br>12.5<br>6.25 | 5<br>5<br>4.5 | 5<br>5<br>5 | 5<br>5<br>5 | 3<br>2<br>2 | -<br>-<br>- |
| 38 | 25<br>12.5<br>6.25 | 5<br>5<br>5 | 5<br>5<br>5 | 5<br>5<br>5 | 5<br>5<br>2 | ++<br>+<br>± |
| 41 | 25<br>12.5<br>6.25 | 5<br>4.8<br>3 | 5<br>5<br>5 | 5<br>5<br>5 | 5<br>5<br>4 | +<br>±<br>- |
| 46 | 25<br>12.5<br>6.25 | 5<br>5<br>5 | 5<br>5<br>5 | 5<br>5<br>5 | 5<br>3.5<br>3 | +<br>-<br>- |
| 48 | 25<br>12.5<br>6.25 | 5<br>4<br>3 | 5<br>5<br>5 | 5<br>5<br>5 | 3<br>2<br>2 | +<br>+<br>- |
| 51 | 25<br>12.5<br>6.25 | 5<br>5<br>4.5 | 5<br>5<br>5 | 5<br>5<br>5 | 5<br>5<br>4 | +<br>-<br>- |
| 52 | 25<br>12.5<br>6.25 | 5<br>5<br>5 | 5<br>5<br>5 | 5<br>5<br>5 | 5<br>5<br>4.5 | ±<br>-<br>- |
| 53 | 25<br>12.5<br>6.25 | 5<br>5<br>5 | 5<br>5<br>5 | 5<br>5<br>5 | 5<br>5<br>4 | ±<br>-<br>- |

| Compound No. | Dosage g/a | Herbicidal activity | | | | Phytotoxicity against paddy rice |
|---|---|---|---|---|---|---|
| | | Barnyard grass | Broad leaf (1) | Umbrella sedge (2) | Arrow head | |
| 54 | 25<br>12.5<br>6.25 | 5<br>5<br>5 | 5<br>5<br>5 | 5<br>5<br>5 | 5<br>5<br>4 | +<br>-<br>- |
| 55 | 25<br>12.5<br>6.25 | 5<br>5<br>5 | 5<br>5<br>5 | 5<br>5<br>5 | 4<br>3<br>2 | -<br>-<br>- |
| 58 | 25<br>12.5<br>6.25 | 5<br>5<br>5 | 5<br>5<br>5 | 5<br>5<br>5 | 5<br>5<br>4.5 | ++<br>+<br>+ |
| 62 | 25<br>12.5<br>6.25 | 5<br>5<br>5 | 5<br>5<br>5 | 5<br>5<br>5 | 5<br>4.5<br>3 | ++<br>±<br>- |
| 65 | 25<br>12.5<br>6.25 | 5<br>5<br>5 | 5<br>5<br>5 | 5<br>5<br>5 | 5<br>5<br>4.5 | ++<br>±<br>± |
| 67 | 25<br>12.5<br>6.25 | 5<br>5<br>5 | 5<br>5<br>5 | 5<br>5<br>5 | 5<br>5<br>4.5 | ++<br>±<br>± |
| 68 | 25<br>12.5<br>6.25 | 5<br>5<br>5 | 5<br>5<br>5 | 5<br>5<br>5 | 2<br>2<br>2 | -<br>-<br>- |
| 69 | 25<br>12.5<br>6.25 | 5<br>5<br>5 | 5<br>5<br>5 | 5<br>5<br>5 | 5<br>5<br>5 | ++<br>+<br>± |
| 70 | 25<br>12.5<br>6.25 | 5<br>5<br>5 | 5<br>5<br>5 | 5<br>5<br>5 | 5<br>5<br>5 | ++<br>+<br>+ |
| 71 | 25<br>12.5<br>6.25 | 5<br>5<br>5 | 5<br>5<br>5 | 5<br>5<br>5 | 5<br>5<br>5 | ++<br>-<br>- |
| 72 | 25<br>12.5<br>6.25 | 5<br>5<br>5 | 5<br>5<br>5 | 5<br>5<br>5 | 5<br>5<br>2 | +<br>-<br>- |
| 73 | 25<br>12.5<br>6.25 | 5<br>5<br>5 | 5<br>5<br>5 | 5<br>5<br>5 | 5<br>5<br>5 | ++<br>+<br>+ |

| Compound No. | Dosage g/a | Harbicidal activity | | | | Phytotoxicity against paddy rice |
|---|---|---|---|---|---|---|
| | | Barnyard grass | Broad leaf (1) | Umbrella sedge (2) | Arrow head | |
| 75 | 25<br>12.5<br>6.25 | 5<br>5<br>5 | 5<br>5<br>5 | 5<br>5<br>5 | 5<br>5<br>5 | +<br>-<br>- |
| 76 | 25<br>12.5<br>6.25 | 5<br>5<br>5 | 5<br>5<br>5 | 5<br>5<br>5 | 5<br>5<br>5 | ±<br>-<br>- |
| 77 | 25<br>12.5<br>6.25 | 5<br>5<br>5 | 5<br>5<br>5 | 5<br>5<br>5 | 5<br>5<br>5 | +<br>±<br>- |
| 78 | 25<br>12.5<br>6.25 | 5<br>5<br>5 | 5<br>5<br>5 | 5<br>5<br>5 | 5<br>5<br>4.5 | +<br>±<br>- |
| 79 | 25<br>12.5<br>6.25 | 5<br>5<br>5 | 5<br>5<br>5 | 5<br>5<br>5 | 5<br>5<br>5 | +<br>-<br>- |
| 80 | 25<br>12.5<br>6.25 | 5<br>5<br>5 | 5<br>5<br>5 | 5<br>5<br>5 | 5<br>5<br>4.5 | +<br>±<br>- |
| 81 | 25<br>12.5<br>6.25 | 5<br>5<br>5 | 5<br>5<br>5 | 5<br>5<br>5 | 5<br>5<br>4 | +<br>+<br>- |
| 82 | 25<br>12.5<br>6.25 | 5<br>5<br>5 | 5<br>5<br>5 | 5<br>5<br>5 | 5<br>5<br>5 | ++<br>+<br>- |
| 83 | 25<br>12.5<br>6.25 | 5<br>5<br>5 | 5<br>5<br>5 | 5<br>5<br>5 | 5<br>5<br>4 | ++<br>+<br>- |
| 84 | 25<br>12.5<br>6.25 | 5<br>5<br>5 | 5<br>5<br>5 | 5<br>5<br>5 | 5<br>5<br>4 | ++<br>+<br>± |
| 85 | 25<br>12.5<br>6.25 | 5<br>5<br>4 | 5<br>5<br>5 | 5<br>5<br>5 | 5<br>3<br>2 | +<br>-<br>- |
| 88 | 25<br>12.5<br>6.25 | 5<br>5<br>5 | 5<br>5<br>5 | 5<br>5<br>5 | 5<br>4<br>3 | +<br>-<br>- |

| Compound No. | Dosage g/a | Harbicidal activity | | | | Phytotoxicity against paddy rice |
|---|---|---|---|---|---|---|
| | | Barnyard grass | Broad leaf (1) | Umbrella sedge (2) | Arrow head | |
| 89 | 25<br>12.5<br>6.25 | 5<br>5<br>5 | 5<br>5<br>5 | 5<br>5<br>5 | 5<br>3<br>3 | +<br>±<br>- |
| 90 | 25<br>12.5 | 5<br>5 | 5<br>5 | 5<br>5 | 5<br>5 | +<br>- |
| 91 | 25<br>12.5 | 5<br>5 | 5<br>5 | 5<br>5 | 5<br>5 | +<br>- |
| 92 | 25<br>12.5 | 5<br>5 | 5<br>5 | 5<br>5 | 4<br>3.5 | +<br>- |
| 111 | 25<br>12.5<br>6.25 | 5<br>5<br>5 | 5<br>5<br>5 | 5<br>5<br>5 | 5<br>5<br>5 | +<br>±<br>- |
| Known compound No. 1 | 25<br>12.5<br>6.25 | 5<br>4<br>2 | 5<br>4<br>3 | 4<br>3<br>1 | 1<br>0<br>0 | -<br>-<br>- |
| Control | 25<br>12.5 | 3<br>1 | 4<br>2 | 3<br>0 | 0<br>0 | +<br>- |

Remarks : (1) Broad leaf : Mixture of barnyard grass, toothcup, false pimpernel, water wort

(2) Umbrella sedge : Umbrella plant

Known compound No. 1

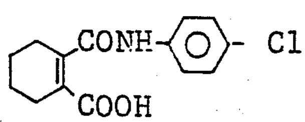

(Japanese Patent Kokai No.44425/1973 Example 3)

Control compound

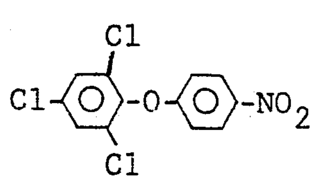

Test Example 2 : Post-emergence treatment in flooded condition

A fixed amount of paddy field soil was filled in each Wagner pot sized 1/5,000 are to provide a condition similar to a paddy field and there was sown a fixed amount of seeds of barnyard grass, monochoria, toothcup, false pimpernel, water wort and umbrella plant.

In addition tubers of arrowhead were buried 1 cm under the surface of soil at the rate of 3 pieces per pot, three 2.5-leaf stage rice seedlings (variety : Nihonbare) were tansplanted from a nursery, the pot was flooded with water 3 cm deep and then placed in a greenhouse.

When the weeds grew to reach 2 to 3-leaf stage, a diluted solution of the compound of the present invention, was applied to the flood at a rate of 12.5 to 50 g of the compound of the present invention per are.

After 30 days from the treatment with the diluted solution, the herbicidal activity was observed and obtained the results as shown in Table 3. The classification basis of the results is the same with Test Example 1.

0097056

Table 3

Test Example 2 : Post-emergence treatment in flooded condition

| Compound No. | Dosage g/a | Herbicidal activity | | | |
|---|---|---|---|---|---|
| | | Barnyard grass | Broad leaf (1) | Umbrella sedge (2) | Arrow-head |
| 3 | 25<br>12.5 | 4<br>3 | 5<br>5 | 5<br>5 | 5<br>5 |
| 6 | 25<br>12.5 | 5<br>5 | 5<br>5 | 5<br>5 | 5<br>5 |
| 9 | 25<br>12.5 | 4<br>3 | 5<br>5 | 5<br>5 | 5<br>5 |
| 13 | 50<br>25 | 5<br>5 | 5<br>5 | 5<br>5 | 5<br>5 |
| 19 | 25<br>12.5 | 5<br>5 | 5<br>5 | 5<br>5 | 5<br>5 |
| 25 | 25<br>12.5 | 4<br>3 | 5<br>5 | 5<br>5 | 4<br>3 |
| 28 | 50<br>25 | 4<br>2 | 5<br>5 | 5<br>5 | 3<br>2 |
| 36 | 25<br>12.5 | 5<br>5 | 5<br>5 | 5<br>5 | 5<br>5 |
| 42 | 25<br>12.5 | 5<br>5 | 5<br>5 | 5<br>5 | 5<br>5 |
| 44 | 25<br>12.5 | 5<br>5 | 5<br>5 | 5<br>5 | 5<br>3 |
| 48 | 25<br>12.5 | 5<br>3 | 5<br>5 | 5<br>5 | 3<br>2 |
| 50 | 25<br>12.5 | 5<br>5 | 5<br>5 | 5<br>5 | 5<br>5 |
| 53 | 25<br>12.5 | 4<br>3 | 5<br>5 | 5<br>5 | 5<br>3 |
| 56 | 25<br>12.5 | 5<br>5 | 5<br>5 | 5<br>5 | 5<br>5 |

| Compound No. | Dosage g/a | Herbicidal activity | | | |
|---|---|---|---|---|---|
| | | Barnyard grass | Broad leaf (1) | Umbrella sedge (2) | Arrow-head |
| 57 | 25<br>12.5 | 5<br>5 | 5<br>5 | 5<br>5 | 5<br>4 |
| 59 | 25<br>12.5 | 5<br>5 | 5<br>5 | 5<br>5 | 5<br>5 |
| 61 | 25<br>12.5 | 5<br>5 | 5<br>5 | 5<br>5 | 5<br>5 |
| 63 | 25<br>12.5 | 5<br>5 | 5<br>5 | 5<br>5 | 5<br>5 |
| 64 | 25<br>12.5 | 5<br>4.5 | 5<br>5 | 5<br>5 | 5<br>5 |
| 65 | 25<br>12.5 | 5<br>4.5 | 5<br>5 | 5<br>5 | 5<br>5 |
| 67 | 25<br>12.5 | 4<br>2 | 5<br>5 | 5<br>5 | 5<br>5 |
| 69 | 25<br>12.5 | 5<br>3 | 5<br>5 | 5<br>5 | 5<br>5 |
| 70 | 25<br>12.5 | 4.5<br>4.5 | 5<br>5 | 5<br>5 | 3<br>3 |
| 71 | 25<br>12.5 | 5<br>5 | 5<br>5 | 5<br>5 | 5<br>5 |
| 73 | 25<br>12.5 | 5<br>5 | 5<br>5 | 5<br>5 | 5<br>5 |
| 74 | 25<br>12.5 | 5<br>5 | 5<br>5 | 5<br>5 | 5<br>5 |
| 75 | 25<br>12.5 | 5<br>3 | 5<br>5 | 5<br>5 | 5<br>5 |
| 76 | 25<br>12.5 | 5<br>5 | 5<br>5 | 5<br>5 | 4<br>3 |
| 77 | 25<br>12.5 | 5<br>5 | 5<br>5 | 5<br>5 | 5<br>2 |
| 78 | 25<br>12.5 | 5<br>5 | 5<br>5 | 5<br>5 | 5<br>5 |
| 79 | 25<br>12.5 | 5<br>5 | 5<br>5 | 5<br>5 | 5<br>5 |
| 80 | 25<br>12.5 | 5<br>5 | 5<br>5 | 5<br>5 | 4<br>5 |

| Compound No. | Dosage g/a | Herbicidal activity | | | |
|---|---|---|---|---|---|
| | | Barnyard grass | Broad leaf (1) | Umbrella sedge (2) | Arrow-head |
| 81 | 25<br>12.5 | 5<br>5 | 5<br>5 | 5<br>5 | 5<br>5 |
| 82 | 50<br>25 | 5<br>5 | 5<br>5 | 5<br>5 | 5<br>5 |
| 83 | 25<br>12.5 | 5<br>3 | 5<br>5 | 5<br>5 | 4<br>3 |
| 84 | 50<br>25 | 4.5<br>3 | 5<br>5 | 5<br>5 | 4<br>3 |
| 86 | 50<br>25 | 4<br>3 | 5<br>5 | 5<br>5 | 4<br>3 |
| 88 | 50<br>25 | 5<br>3 | 5<br>5 | 5<br>5 | 5<br>4 |
| 89 | 25<br>12.5 | 4.5<br>3 | 5<br>5 | 5<br>5 | 4.5<br>3 |
| 91 | 25<br>12.5 | 4.5<br>4 | 5<br>5 | 5<br>5 | 3<br>2 |
| 93 | 25<br>12.5 | 5<br>4 | 5<br>5 | 5<br>5 | 4<br>3 |
| 111 | 25<br>12.5 | 5<br>5 | 5<br>5 | 5<br>5 | 5<br>5 |
| Known compound No. 1 | 25<br>12.5 | 3<br>0 | 5<br>5 | 5<br>5 | 2<br>0 |
| Control A | 50<br>25 | 1<br>0 | 1<br>0 | 0<br>0 | 0<br>0 |

Remarks (1)<br>(2) }the same as in Test Example 1

Known compound No. 1<br>Control A }the same as in Test Example 1

Test Example 3: Test on perennial weeds in a paddy field

Wagner pots sized 1/5,000 are were filled with a fixed amount of paddy field soil to provide a condition similar to a paddy field and there was sown a fixed amount of seeds of bulrush. In addition tubers of mizugayatsuri and water chestnut were buried 3 cm under the surface of soil at the rate of 3 pieces per pot and then the pot was flooded with water 3 cm deep.

The pre-emergence treatment was conducted on the second day after seeds and tubers of the weeds were put into soil, while the post-emergence treatment was effected at 2-leaf stage of bulrush, 2 to 3-leaf stage of mizugayatsuri and the time when water chestnut grew 5 to 6 cm high, at each time a diluted solution of the compound of the present invention was applied to the flood at a rate of 12.5 to 50 g of the compound of the present invention per are.

The herbicidal activity was observed on 30th day after each treatment and the test results are shown in Table 4. The judging standard of the results is the same as Test Example 1.

Table 4

Test Example 3: Test on perennial weed in paddy field

| Compound No. | Dosage g/a | Pre-emergence treatment | | | Post-emergence treatment | | |
|---|---|---|---|---|---|---|---|
| | | Bulrush | Mizugayatsuri | Water chesnut | Bulrush | Mizugayatsuri | Water chesnut |
| 3 | 50<br>25<br>12.5 | 5<br>5<br>5 | 5<br>5<br>5 | 5<br>5<br>5 | 5<br>5<br>5 | 5<br>5<br>4 | 5<br>4<br>3 |
| 9 | 50<br>25<br>12.5 | 5<br>5<br>5 | 5<br>5<br>5 | 5<br>5<br>5 | 5<br>3<br>3 | 5<br>4<br>3 | 4<br>3<br>2 |
| 19 | 50<br>25<br>12.5 | 5<br>5<br>5 | 5<br>5<br>5 | 5<br>4.5<br>4 | 5<br>5<br>5 | 5<br>5<br>5 | 5<br>4.5<br>4 |
| 31 | 50<br>25<br>12.5 | 5<br>5<br>5 | 5<br>5<br>5 | 5<br>4<br>3 | 5<br>5<br>4 | 4<br>3<br>3 | 4<br>3<br>2 |
| 45 | 50<br>25<br>12.5 | 5<br>5<br>5 | 5<br>5<br>5 | 5<br>5<br>5 | 5<br>5<br>5 | 5<br>5<br>5 | 5<br>5<br>3 |
| 50 | 50<br>25<br>12.5 | 5<br>5<br>5 | 5<br>5<br>5 | 5<br>5<br>5 | 5<br>5<br>5 | 5<br>5<br>4 | 5<br>4<br>3 |
| 61 | 50<br>25<br>12.5 | 5<br>5<br>5 | 5<br>5<br>5 | 5<br>5<br>5 | 5<br>5<br>5 | 5<br>5<br>5 | 5<br>5<br>4.5 |
| 65 | 50<br>25<br>12.5 | 5<br>5<br>5 | 5<br>5<br>5 | 5<br>5<br>5 | 5<br>5<br>5 | 5<br>5<br>4.5 | 5<br>5<br>4.5 |
| 71 | 50<br>25<br>12.5 | 5<br>5<br>5 | 5<br>5<br>5 | 5<br>5<br>5 | 5<br>5<br>5 | 5<br>5<br>5 | 5<br>5<br>5 |
| 111 | 50<br>25<br>12.5 | 5<br>5<br>5 | 5<br>5<br>5 | 5<br>5<br>5 | 5<br>5<br>5 | 5<br>5<br>5 | 5<br>5<br>4 |

As seen in the results of Test example 1, 2 and 3, the compounds of the present invention showed remarkable herbicidal effect against the annual and perennial weeds in paddy fields in pre- and post emergence treatment.

Furthermore, it was found that the compound of the present invention showed only little phytotoxicity in pre- and post transplantation treatment.

Then the Test examples in field are shown as follows.

Test Example 4 : Pre-emergence soil surface treatment

A fixed amount of field soil was filled in a round plastic case 8 cm across and 8 cm deep, and a fixed amount of seeds of crabgrass, foxtail, pigweed, lamb's-quarters was sown followed by covering them with soil 0.5 to 1 cm thick. Then immediately a diluted solution of the compound of the present invention was applied to treat the whole surface of soil in case at a rate of 12.5 to 25 g of the compound of the present invention per are.

After the treatment the cultivation was done in a greenhouse and the herbicidal activity was observed on the 20th day. The test was carried out on 2-replication system and each average value was obtained The judging standard of the results is the same as Test Example 1. The test results are shown in Table 5.

Table 5

Test Example 4: Pre-emergence soil surface treatment

| Compound No. | Dosage g/a | Herbicidal activity | | | |
|---|---|---|---|---|---|
| | | Foxtail | Crabgrass | Pigweed | Buckweat |
| 14 | 25<br>12.5 | 4.5<br>3.5 | 5<br>5 | 5<br>5 | 5<br>5 |
| 15 | 25<br>12.5 | 5<br>4 | 5<br>5 | 5<br>5 | 5<br>5 |
| 18 | 25<br>12.5 | 4.5<br>3 | 5<br>5 | 5<br>5 | 5<br>5 |
| 21 | 25<br>12.5 | 3.5<br>3 | 4.5<br>4 | 5<br>5 | 5<br>5 |
| 22 | 25<br>12.5 | 3<br>2 | 4<br>3 | 5<br>5 | 5<br>5 |
| 24 | 25<br>12.5 | 4<br>3 | 5<br>4 | 5<br>5 | 5<br>5 |
| 32 | 25<br>12.5 | 3<br>2 | 5<br>4.5 | 5<br>5 | 5<br>5 |
| 42 | 25<br>12.5 | 3.5<br>3 | 5<br>5 | 5<br>5 | 5<br>5 |
| 43 | 25<br>12.5 | 5<br>5 | 5<br>5 | 5<br>5 | 5<br>5 |
| 44 | 25<br>12.5 | 5<br>5 | 5<br>5 | 5<br>5 | 5<br>5 |
| 46 | 25<br>12.5 | 5<br>5 | 5<br>5 | 5<br>5 | 5<br>5 |
| 47 | 25<br>12.5 | 5<br>5 | 5<br>5 | 5<br>5 | 5<br>5 |
| 52 | 25<br>12.5 | 3<br>2 | 5<br>3 | 5<br>5 | 5<br>5 |

| Compound No. | Dosage g/a | Herbicidal activity | | | |
|---|---|---|---|---|---|
| | | Foxtail | Crabgrass | Pigweed | Buckweat |
| 57 | 25<br>12.5 | 5<br>3 | 5<br>5 | 5<br>5 | 5<br>5 |
| 58 | 25<br>12.5 | 5<br>5 | 5<br>5 | 5<br>5 | 5<br>5 |
| 59 | 25<br>12.5 | 5<br>5 | 5<br>5 | 5<br>5 | 5<br>5 |
| 61 | 25<br>12.5 | 4.5<br>4 | 5<br>4 | 5<br>5 | 5<br>5 |
| 63 | 25<br>12.5 | 5<br>5 | 5<br>5 | 5<br>5 | 5<br>5 |
| 64 | 25<br>12.5 | 5<br>5 | 5<br>5 | 5<br>5 | 5<br>5 |
| 65 | 25<br>12.5 | 5<br>5 | 5<br>5 | 5<br>5 | 5<br>5 |
| 67 | 25<br>12.5 | 5<br>4.5 | 5<br>5 | 5<br>5 | 5<br>5 |
| 69 | 25<br>12.5 | 5<br>4.5 | 5<br>5 | 5<br>5 | 5<br>5 |
| 70 | 25<br>12.5 | 5<br>4.5 | 5<br>5 | 5<br>5 | 5<br>5 |
| 73 | 25<br>12.5 | 4<br>3 | 4.5<br>3 | 5<br>5 | 5<br>5 |
| 74 | 25<br>12.5 | 4<br>3 | 4<br>3 | 5<br>5 | 5<br>5 |
| 75 | 25<br>12.5 | 4.5<br>4 | 5<br>5 | 5<br>5 | 5<br>5 |
| 76 | 25<br>12.5 | 5<br>5 | 5<br>5 | 5<br>5 | 5<br>5 |
| 77 | 25<br>12.5 | 5<br>5 | 5<br>5 | 5<br>5 | 5<br>5 |
| 78 | 25<br>12.5 | 5<br>5 | 5<br>5 | 5<br>5 | 5<br>5 |
| 79 | 25<br>12.5 | 5<br>5 | 5<br>5 | 5<br>5 | 5<br>5 |
| 80 | 25<br>12.5 | 5<br>5 | 5<br>5 | 5<br>5 | 5<br>5 |

| Compound No. | Dosage g/a | Herbicidal activity | | | |
|---|---|---|---|---|---|
| | | Foxtail | Crabgrass | Pigweed | Buckweat |
| 81 | 25<br>12.5 | 5<br>5 | 5<br>5 | 5<br>5 | 5<br>5 |
| 82 | 25<br>12.5 | 5<br>5 | 5<br>5 | 5<br>5 | 5<br>5 |
| 83 | 25<br>12.5 | 5<br>5 | 5<br>5 | 5<br>5 | 5<br>5 |
| 86 | 25<br>12.5 | 4.5<br>4.5 | 4.5<br>4 | 5<br>5 | 5<br>5 |
| 88 | 25<br>12.5 | 5<br>5 | 5<br>5 | 5<br>5 | 5<br>5 |
| 91 | 25<br>12.5 | 5<br>5 | 5<br>5 | 5<br>5 | 5<br>5 |
| 111 | 25<br>12.5 | 5<br>5 | 5<br>5 | 5<br>5 | 5<br>5 |
| Known compound No. 1 | 25<br>12.5 | 2<br>1 | 2<br>0 | 5<br>5 | 4<br>2 |
| Control B | 25<br>12.5 | 4<br>2 | 4.5<br>3 | 5<br>5 | 5<br>5 |

Known compound No. 1

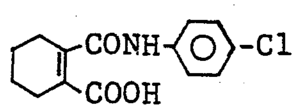

Control B

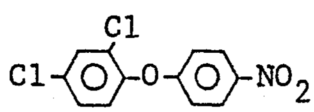

Test Example 5 : Post-emergence treatment

A fixed amount of field soil was filled in a round plastic case 8 cm across and 8 cm deep, and a fixed amount of seeds of foxtail, pigweed was sown. When they grew up to 3 to 4-leaf stage, a wettable powder containing the compound of the present invention was sprayed on the body of plants after diluting it at a rate of 12.5, 25 or 50 g of active ingredient per are.

The test was conducted on 2-replication system. Twenty days after the treatment the test results were observed on the same judging standard and the results are shown in Table 6.

Table 6

Test Example 5 : Post-emergence treatment

| Compound No. | Dosage g/a | Herbicidal activity | | Compound No. | Dosage g/a | Herbicidal activity | |
|---|---|---|---|---|---|---|---|
| | | Foxtail | Pigweed | | | Foxtail | Pigweed |
| 4 | 50<br>25<br>12.5 | 5<br>4<br>3 | 5<br>5<br>5 | 62 | 50<br>25<br>12.5 | 5<br>4.5<br>3.5 | 5<br>5<br>5 |
| 12 | 50<br>25<br>12.5 | 5<br>5<br>4 | 5<br>5<br>5 | 63 | 50<br>25<br>12.5 | 5<br>4<br>3 | 5<br>5<br>5 |
| 21 | 50<br>25<br>12.5 | 5<br>3.5<br>3 | 5<br>5<br>5 | 66 | 50<br>25<br>12.5 | 4<br>3<br>2 | 5<br>5<br>5 |
| 34 | 50<br>25<br>12.5 | 4.5<br>4<br>2 | 5<br>5<br>4.5 | 67 | 50<br>25<br>12.5 | 3.5<br>3<br>2 | 5<br>5<br>4.5 |
| 41 | 50<br>25<br>12.5 | 5<br>4.5<br>3.5 | 5<br>5<br>3.5 | 69 | 50<br>25<br>12.5 | 5<br>4<br>3 | 5<br>5<br>5 |
| 43 | 50<br>25<br>12.5 | 5<br>3<br>2 | 5<br>5<br>5 | 70 | 50<br>25<br>12.5 | 5<br>4<br>3 | 5<br>5<br>5 |
| 56 | 50<br>25<br>12.5 | 5<br>5<br>4 | 5<br>5<br>5 | 71 | 50<br>25<br>12.5 | 5<br>5<br>4 | 5<br>5<br>5 |
| 57 | 50<br>25<br>12.5 | 5<br>4<br>3 | 5<br>5<br>5 | 72 | 50<br>25<br>12.5 | 4<br>3<br>2 | 5<br>5<br>5 |
| 58 | 50<br>25<br>12.5 | 5<br>5<br>4.5 | 5<br>5<br>5 | 74 | 50<br>25<br>12.5 | 4<br>2.5<br>2 | 5<br>5<br>5 |
| 59 | 50<br>25<br>12.5 | 5<br>5<br>5 | 5<br>5<br>5 | 75 | 50<br>25<br>12.5 | 4<br>3<br>2 | 5<br>5<br>5 |
| 76 | 50<br>25<br>12.5 | 5<br>5<br>4.5 | 5<br>5<br>5 | 93 | 50<br>25<br>12.5 | 3.5<br>3<br>2 | 5<br>5<br>5 |

| Compound No. | Dosage g/a | Herbicidal activity | |
|---|---|---|---|
| | | Foxtail | Pigweed |
| 77 | 50<br>25<br>12.5 | 5<br>5<br>5 | 5<br>5<br>5 |
| 78 | 50<br>25<br>12.5 | 5<br>5<br>5 | 5<br>5<br>5 |
| 79 | 50<br>25<br>12.5 | 5<br>5<br>5 | 5<br>5<br>5 |
| 80 | 50<br>25<br>12.5 | 5<br>5<br>5 | 5<br>5<br>5 |
| 81 | 50<br>25<br>12.5 | 5<br>4.5<br>4.5 | 5<br>5<br>5 |
| 82 | 50<br>25<br>12.5 | 5<br>3<br>2 | 5<br>5<br>5 |
| 83 | 50<br>25<br>12.5 | 5<br>5<br>3 | 5<br>5<br>5 |
| 89 | 50<br>25<br>12.5 | 5<br>4<br>3 | 5<br>5<br>5 |
| 91 | 50<br>25<br>12.5 | 4<br>3<br>2 | 5<br>5<br>5 |

| Compound No. | Dosage g/a | Herbicidal activity | |
|---|---|---|---|
| | | Foxtail | Pigweed |
| 111 | 50<br>25<br>12.5 | 5<br>5<br>5 | 5<br>5<br>5 |
| Known compound No. 1 | 50<br>25<br>12.5 | 0<br>0<br>0 | 5<br>4<br>3 |
| Control B | 50<br>25<br>12.5 | 5<br>3<br>2 | 5<br>5<br>4.5 |

Test Example 6 : Phytotoxicity against crops

A fixed amount of field soil was filled in a plastic vessell sized 23 cm x 4.5 cm x 12.5 cm and a fixed amount of seeds of soybean, cotton, corn, wheat, sunflower and rice was sown followed by 3-cm thick covering with soil.

Then immediately a diluted solution of the compound of the present invention was sprayed on the soil surface with a small sprayer at the rate of 25 to 50 g of the compound of the present invention.

After the treatment the crops were grown in a greenhouse and 20 days later the degree of phytotoxicity against each crop was observed. The test was carried out on 2-replication system and each average value was obtained.

The judging standard of test results is the same as Test Example 1 and the result are shown in Table 7.

Table 7

Test Example 6 : Phytotoxicity against crops

| Compound No. | Dosage g/a | Phytotoxicity against crops | | | | | |
|---|---|---|---|---|---|---|---|
| | | Soybean | Cotton | Corn | Wheat | Rice | Sunflower |
| 15 | 50 | - | - | - | - | - | - |
| | 25 | - | - | - | - | - | - |
| 18 | 50 | | - | - | - | - | - |
| | 25 | - | - | - | - | - | - |
| 24 | 50 | - | - | - | - | - | - |
| | 25 | - | - | - | - | - | - |
| 43 | 50 | - | - | - | - | - | - |
| | 25 | - | - | - | - | - | - |
| 46 | 50 | - | - | - | - | - | - |
| | 25 | - | - | - | - | - | - |
| 47 | 50 | - | - | - | - | - | - |
| | 25 | - | - | - | - | - | - |
| 58 | 50 | - | - | - | - | - | - |
| | 25 | - | - | - | - | - | - |
| 59 | 50 | | - | - | - | - | - |
| | 25 | - | - | - | - | - | - |
| 63 | 50 | - | - | - | - | - | - |
| | 25 | - | - | - | - | - | - |
| 64 | 50 | - | - | - | - | - | - |
| | 25 | - | - | - | - | - | - |
| 70 | 50 | - | - | - | - | - | - |
| | 25 | - | - | - | - | - | - |
| 73 | 50 | - | - | - | - | - | - |
| | 25 | - | - | - | - | - | - |
| 75 | 50 | | - | | | - | - |
| | 25 | - | - | - | - | - | - |
| 76 | 50 | - | ++ | + | + | + | - |
| | 25 | - | + | - | - | - | - |
| 78 | 50 | - | + | - | - | + | - |
| | 25 | - | - | - | - | - | - |
| 80 | 50 | + | + | - | + | + | - |
| | 25 | - | - | - | - | - | - |

| Compound No. | Dosage g/a | Phytotoxicity against crops | | | | | |
|---|---|---|---|---|---|---|---|
| | | Soybean | Cotton | Corn | Wheat | Rice | Sunflower |
| 81 | 50<br>25 | +<br>- | ++<br>- | +<br>- | -<br>- | +<br>- | -<br>- |
| 82 | 50<br>25 | +<br>- | ++<br>- | -<br>- | -<br>- | +<br>- | -<br>- |
| 83 | 50<br>25 | +<br>- | +<br>- | -<br>- | -<br>- | +<br>- | -<br>- |
| 84 | 50<br>25 | -<br>- | +<br>- | -<br>- | -<br>- | +<br>- | -<br>- |
| 86 | 50<br>25 | +<br>- | ++<br>- | -<br>- | -<br>- | +<br>- | -<br>- |
| 88 | 50<br>25 | +<br>- | +<br>- | +<br>- | -<br>- | +<br>- | -<br>- |
| 92 | 50<br>25 | +<br>- | ++<br>- | +<br>- | +<br>- | -<br>- | -<br>- |
| 93 | 50<br>25 | -<br>- | +<br>- | +<br>- | -<br>- | +<br>- | -<br>- |
| 111 | 50<br>25 | -<br>- | +<br>- | -<br>- | -<br>- | -<br>- | -<br>- |
| Known compound No. 1 | 50<br>25 | -<br>- | -<br>- | -<br>- | -<br>- | -<br>- | -<br>- |
| Control B | 50<br>25 | ++<br>+ | +++<br>++ | +++<br>++ | +++<br>++ | ++<br>+ | +<br>- |

As obvious from the results of Test Examples 4 and 5, the compound of the present invention proves to show very good herbicidal activity both in pre-emergence and post-emergence treatments of main weeds in the field. On the other hand, it is clear from the results of Test Example 6 that the compound of the present invention has no phytotoxicity against crops and is a suitable herbicide for use in farmlands.

CLAIMS

1. An N-substituted-3,4,5,6-tetrahydrophthalamic acid derivative of the formula:

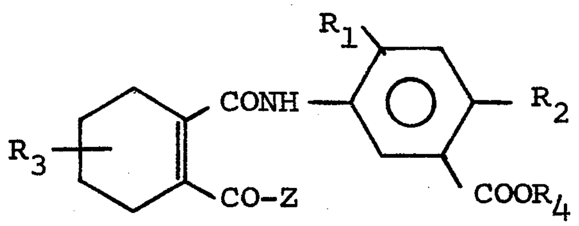

(I)

wherein $R_1$ is hydrogen or halogen; $R_2$ is halogen; $R_3$ is hydrogen or $C_1$-$C_4$ alkyl; $R_4$ is hydrogen or $C_1$-$C_8$- alkyl which may be substituted by halogen or $C_1$-$C_4$ alkoxy; and Z is hydroxy, $C_1$-$C_8$-primary or secondary alkylamino, $C_2$-$C_5$-alkenyl amino, $C_3$-$C_7$ cycloalkylamino, furfurylamino, benzylamino, morpholino which may be substituted by methyl, or $C_2$-$C_4$ alkylamino which may be substituted by phenyl, $C_1$ or $C_2$-alkylamino, $C_1$-$C_4$-alkoxy, $C_1$-$C_4$-alkylthio or cyano; or an alkali metal salt thereof.

2. A compound according to claim 1 wherein $R_1$ is chloro or fluoro, $R_2$ is chloro or bromo, $R_3$ is hydrogen or methyl, $R_4$ is $C_2$ or $C_3$-alkyl and Z is hydroxy, $C_1$-$C_3$-alkylamino or $C_1$-$C_3$-alkoxy-$C_2$ or $C_3$-alkylamino.

3. A compound of claim 2 wherein $R_1$ is fluoro, $R_2$ is chloro or bromo, $R_3$ is hydrogen, $R_4$ is isopropyl and Z is $C_1$-$C_3$-alkylamino or $C_1$-$C_3$-alkoxy-$C_2$ or $C_3$-alkylamino.

4. A compound according to claim 3 of the formula:

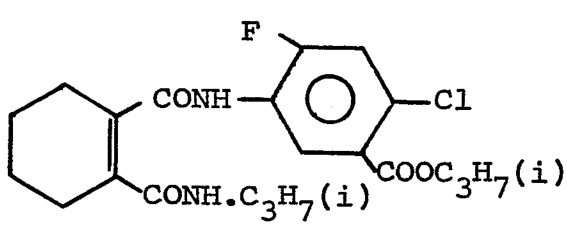

wherein $C_3H_7$(i) represents isopropyl.

5. A process for the preparation of an N-substituted-3,4,5,6-tetrahydrophthalamic acid derivative of the formula (4):

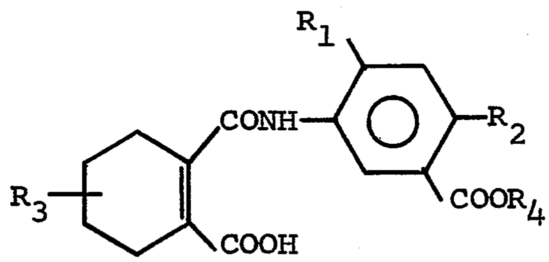

(4)

wherein $R_1$,$R_2$, $R_3$ and $R_4$ are as defined in claim 1, or an alkali metal salt thereof, which process comprises reacting a 3,4,5,6-tetrahydrophthalic anhydride of the formula (2):

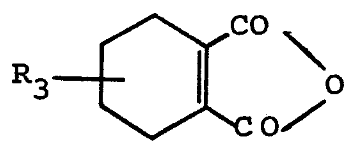

(2)

wherein $R_3$ is as defined above, with a m-aminobenzoic acid derivative of the formula (3):

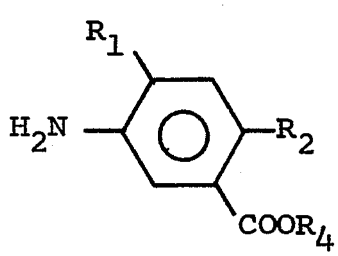

(3)

wherein $R_1$,$R_2$ and $R_4$ are as defined above, and, if desired, converting the resulting compound of formula (4) into an alkali metal salt thereof.

6. A process for the preparation of an N-substituted-3,4,5,6-tetrahydrophthalamic acid derivative of the formula (8):

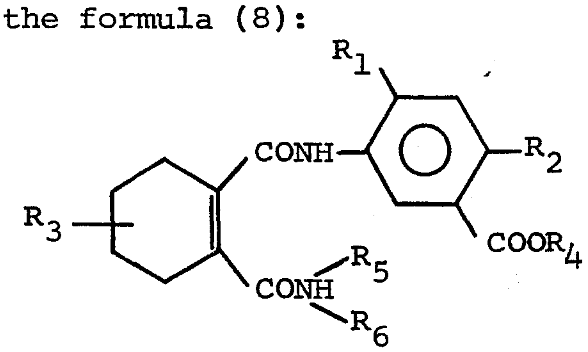

(8)

wherein $R_1$,$R_2$,$R_3$ and $R_4$ are as defined in claim 1, $R_5$ is hydrogen or $C_1$-$C_8$-alkyl and $R_6$ is hydrogen, $C_1$-$C_8$-alkyl, $C_2$-$C_5$-alkenyl, $C_3$-$C_7$-cycloalkyl, furfuryl, benzyl or $C_2$-$C_4$-alkyl which may be substituted by phenyl, $C_1$ or $C_2$-alkylamino, $C_1$-$C_4$-alkoxy, $C_1$-$C_4$-alkylthio or cyano or $R_5$ and $R_6$ together with the nitrogen atom to which they are attached form a morpholino group which may be substituted by methyl, which process comprises reacting a compound of the formula (5):

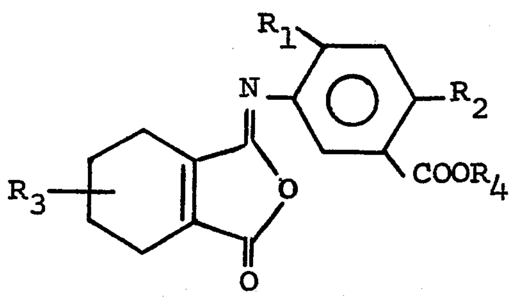

(5)

wherein $R_1$,$R_2$,$R_3$ and $R_4$ are as defined above, with a compound of the formula (7):

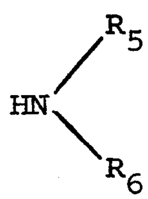

(7)

wherein $R_5$ and $R_6$ are as defined above, in an aqueous or organic solvent.

7. A process for the preparation of an N-substituted-3,4,5,6-tetrahydrophthalamic acid derivative of the formula (8):

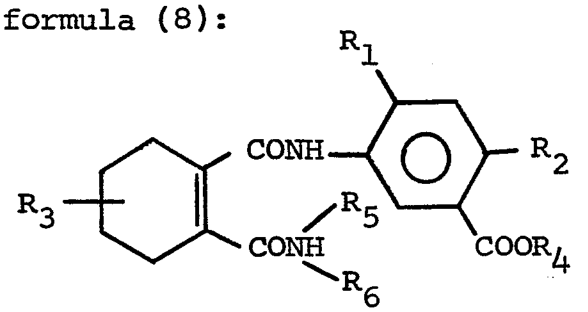

(8)

wherein $R_1$,$R_2$,$R_3$ and $R_4$ are as defined in claim 1 and $R_5$ and $R_6$ are as defined in claim 6, which process comprises reacting a compound of the formula (6):

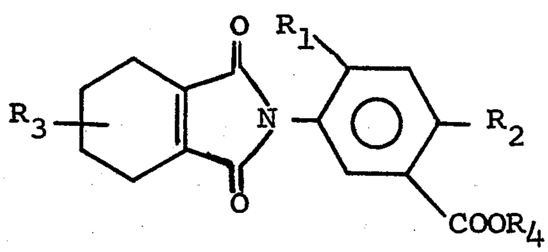

(6)

wherein $R_1$,$R_2$,$R_3$ and $R_4$ are as defined above, with a compound of the formula (7):

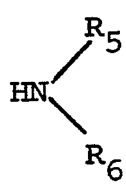

(7)

wherein $R_5$ and $R_6$ are as defined above, in an aqueous or organic solvent.

8. A process according to claim 6 or 7 wherein the compound of formula (5) or (6) has been obtained by heating a compound of formula (4) as defined in claim 5 in the presence of a dehydrating agent.

9. A herbicidal composition which comprises from 0.5 to 95 weight % of an N-substituted-3,4,5,6-tetrahydrophthalamic acid derivative of the formula (1) or an alkali metal salt thereof as claimed in any one of claims 1 to 4 or which has been produced by a process as claimed in any

one of claims 5 to 8, and from 5 to 99.5 weight % of adjuvant(s).

10. A method of controlling the growth of weeds at a locus, which method comprises applying to the locus a herbicidally effective amount of a derivative of N-substituted-3,4,5,6-tetrahydrophthalamic acid of the formula (1) or an alkali metal salt thereof as claimed in any one of claims 1 to 4 or which has been produced by a process as claimed in any one of claims 5 to 8.